**Europäisches Patentamt**

**European Patent Office**

**Office européen des brevets**

(11) Veröffentlichungsnummer: **0 012 412**
**B1**

(12) **EUROPÄISCHE PATENTSCHRIFT**

(45) Veröffentlichungstag der Patentschrift:
02.09.81

(21) Anmeldenummer: **79105042.0**

(22) Anmeldetag: **10.12.79**

(51) Int. Cl.³: **C 07 C 79/37,** C 07 C 76/06

(54) Verfahren und Vorrichtung zur Gewinnung von trockenem 1,5- und/oder 1,8-Dinitroanthrachinon aus Suspensionen, die festes 1,5- und/oder 1,8-Dinitroanthrachinon und flüssiges Nitrobenzol enthalten.

(30) Priorität: **16.12.78 DE 2854427**

(43) Veröffentlichungstag der Anmeldung:
**25.06.80 Patentblatt 80/13**

(45) Bekanntmachung des Hinweises auf die Patenterteilung:
**02.09.81 Patentblatt 81/35**

(84) Benannte Vertragsstaaten:
**BE CH DE FR GB**

(56) Entgegenhaltungen:
**DE-A-2 637 733**
**DE-A-2 654 648**

(73) Patentinhaber: **BAYER AG, Zentralbereich Patente, Marken und Lizenzen, D-5090 Leverkusen 1, Bayerwerk (DE)**

(72) Erfinder: **Schaefer, Axel, Hittorf.Strasse 10, D-5300 Bonn (DE)**
Erfinder: **Motika, Werner, Paul-Klee-Strasse 62, D-5090 Leverkusen (DE)**
Erfinder: **Bender, Wolfgang, Am Jungholzkamp 9, D-5093 Burscheid (DE)**
Erfinder: **Todt, Jörn, Walter-Flex-Strasse 22, D-5090 Leverkusen (DE)**
Erfinder: **Oels, Udo, Bethel-Strasse 22, D-4150 Krefeld (DE)**

## Verfahren und Vorrichtung zur Gewinnung von trockenem 1,5- und/oder 1,8-Dinitroanthrachinon aus Suspensionen, die festes 1,5- und/oder 1,8-Dinitroanthrachinon und flüssiges Nitrobenzol enthalten

In Ullmanns Enzyklopädie der technischen Chemie, 3. Auflage, 3. Band, S. 675 (1953), ist beschrieben, daß bei der Dinitrierung von Anthrachinon schwer trennbare Gemische erhalten werden, die je ca. 40% 1,5- und 1,8- neben ca. 20% 1,6- und 1,7-Dinitroanthrachinon enthalten. Es sind verschiedene Verfahren beschrieben worden, gemäß denen aus rohen Dinitroanthrachinongemischen 1,5- und/oder 1,8-Dinitroanthrachinon mittels Nitrobenzol abgetrennt werden kann (siehe z. B. DE-OS 22 48 704, 25 24 747, 26 37 733 und JP-OS 49-76851). Bei diesen Verfahren erhält man bei erhöhter Temperatur Suspensionen von 1,5- und/oder 1,8-Dinitroanthrachinon in Nitrobenzol. Aus diesen Suspensionen muß dann das Nitrobenzol entfernt werden, um trockenes 1,5- und/oder 1,8-Dinitroanthrachinon zu erhalten.

Es wurde nun ein Verfahren zur Gewinnung von trockenem 1,5- und/oder 1,8-Dinitroanthrachinon aus einer Suspension, die festes 1,5- und/oder 1,8-Dinitroanthrachinon und flüssiges Nitrobenzol enthält, gefunden, das dadurch gekennzeichnet ist, daß man in einer ersten Stufe bei erhöhter Temperatur festes 1,5- und/oder 1,8-Dinitroanthrachinon mit einem Nitrobenzolgehalt von weniger als 30 Gew.-% mechanisch aus der Suspension abtrennt und in einer zweiten Stufe aus dem abgetrennten 1,5- und/oder 1,8-Dinitroanthrachinon restliches Nitrobenzol durch Druckerniedrigung bei sinkender Temperatur entfernt.

In das erfindungsgemäße Verfahren können beliebige Suspensionen eingesetzt werden, die flüssiges Nitrobenzol und festes 1,5-Dinitroanthrachinon oder festes 1,8-Dinitroanthrachinon oder festes 1,5- und 1,8-Dinitroanthrachinon enthalten. Vorzugsweise enthalten diese Suspensionen 2 bis 200 g, insbesondere 5 bis 150 g festes 1,5- und/oder 1,8-Dinitroanthrachinon, jeweils bezogen auf 1 kg flüssiges Nitrobenzol. In gelöster Form können im Nitrobenzol weiteres 1,5- und/oder 1,8-Dinitroanthrachinon sowie weitere Verbindungen enthalten sein, die bei der Nitrierung von Anthrachinon oder Mononitroanthrachinonen entstehen. Geeignete Suspensionen können beispielsweise bei der Aufarbeitung von Dinitroanthrachinon-Gemischen entsprechend der DE-OS 2 637 733 erhalten werden.

Erfindungsgemäß wird aus der eingesetzten Suspension zunächst ein Teil des Nitrobenzols mechanisch abgetrennt. Hierfür können übliche mechanische Trennvorrichtungen verwendet werden, beispielsweise Filter oder Zentrifugen, insbesondere Trommelfilter oder Schneckenzentrifugen. Für die mechanische Abtrennung von Nitrobenzol aus festem 1,5-Dinitroanthrachinon enthaltenden Suspensionen wird vorzugsweise ein Drucktrommelfilter und für die mechanische Abtrennung von Nitrobenzol aus festem 1,8-Dinitroanthrachinon enthaltenden Suspensionen vorzugsweise eine Vollmantel-Schneckenzentrifuge verwendet.

Die mechanische Trennung erfolgt erfindungsgemäß bei erhöhter Temperatur. Geeignete Temperaturen sind beispielsweise solche im Bereich 80 bis 150°C. Vorzugsweise wird die mechanische Trennung bei Temperaturen im Bereich 90 bis 130°C durchgeführt. Da bei den bekannten Verfahren zur Abtrennung von 1,5- und/oder 1,8-Dinitroanthrachinon aus Nitriergemischen mittels Nitrobenzol Suspensionen mit Temperaturen in den angegebenen Bereichen anfallen, können diese Suspensionen normalerweise ohne Kühlung oder Erwärmung direkt in das erfindungsgemäße Verfahren eingesetzt werden.

Es ist vorteilhaft, die mechanische Trennung bei konstanter Temperatur vorzunehmen. Die Vorrichtung für die mechanische Trennung ist deshalb vorzugsweise beheizbar ausgerüstet, um Wärmeverluste, z. B. durch Abstrahlung, ausgleichen zu können.

Die mechanische Trennvorrichtung kann auch so ausgerüstet sein, daß darin noch eine Wäsche des festen 1,5- und/oder 1,8-Dinitroanthrachinons mit Nitrobenzol vorgenommen werden kann.

Die mechanische Abtrennung von Nitrobenzol wird so durchgeführt, daß das zurückbleibende, feste 1,5- und/oder 1,8-Dinitroanthrachinon weniger als 30 Gew.-%, vorzugsweise weniger als 15 Gew.-% Nitrobenzol enthält. Die in der mechanischen Trennvorrichtung abgetrennte flüssige Phase und gegebenenfalls die Waschflüssigkeit werden entfernt. Daraus können gegebenenfalls die gelösten Bestandteile ganz oder teilweise abgetrennt werden, beispielsweise durch Temperaturerniedrigung oder Eindampfung.

Aus dem in der mechanischen Trennvorrichtung abgetrennten festen 1,5- und/oder 1,8-Dinitroanthrachinon, das noch restliches Nitrobenzol enthält, wird nun in der zweiten Stufe restliches Nitrobenzol durch Druckerniedrigung bei sinkender Temperatur entfernt. Man kann hier so verfahren, daß man das Produkt aus der ersten Stufe bei erhöhter Temperatur in einen evakuierbaren Behälter einbringt und diesen ohne äußere Wärmezufuhr evakuiert. Dabei verdampft dem 1,5- und/oder 1,8-Dinitroanthrachinon noch anhaftendes Nitrobenzol unter Abkühlung des 1,5- und/oder 1,8-Dinitroanthrachinons. Die Trocknung ist um so schneller und vollständiger, je schneller und tiefer der Druck im evakuierbaren Behälter erniedrigt wird. Im allgemeinen ist es ausreichend, wenn der Druck auf 0,2 bis 30 mbar erniedrigt wird. Vorzugsweise wird der Druck auf 1 bis 20 mbar erniedrigt. Bei diesen Drücken ist es noch ohne besonderen Aufwand möglich, das verdampfte Nitrobenzol vor der Vakuumpumpe aufzufangen, z. B. durch Kondensation.

Die Temperatur, mit der das Produkt vor der Druckerniedrigung vorliegt, ist normalerweise die gleiche, mit der das Produkt bei der mechanischen Abtrennung von Nitrobenzol anfällt. In manchen Fällen kann es jedoch vorteilhaft sein, das Produkt vor der Erniedrigung des Druckes auf eine höhere Temperatur zu bringen. Beispielsweise ist dies vorteilhaft, wenn die Restfeuchte vor der Druckerniedrigung relativ hoch, die Druckerniedrigung relativ gering und/oder die Temperatur vor der Druckerniedrigung relativ gering ist.

Sofern eine Temperaturerhöhung vorgenommen wird, erfolgt diese maximal bis auf einige Grade unterhalb des Siedepunktes von Nitrobenzol bei Normaldruck. Beispielsweise kann man das Produkt aus der mechanischen Abtrennung von Nitrobenzol mit einer Temperatur von 80 bis 205° C oder 90 bis 190° C in die zweite Stufe einbringen. Besonders bevorzugt wird das Produkt aus der mechanischen Abtrennung von Nitrobenzol mit der Temperatur in die zweite Stufe des Verfahrens eingebracht, mit der es nach der mechanischen Abtrennung von Nitrobenzol vorliegt.

Während der Druckerniedrigung sinkt die Temperatur des festen 1,5- und/oder 1,8-Dinitroanthrachinons in dem Maße ab, wie Nitrobenzol verdampft. Die Trocknung erfolgt im allgemeinen auf Restgehalte an Nitrobenzol von unter 1 Gew.-%, vorzugsweise von unter 0,5 Gew.-%. Das getrocknete Produkt weist dann im allgemeinen eine Temperatur im Bereich von 15 bis 130° C, vorzugsweise 40 bis 95° C, auf. Das getrocknete Produkt kann dann, gegebenenfalls nach einer Kühlung, abgepackt, gelagert oder direkt zu weiteren Umsetzungen verwendet werden.

Ein evakuierbarer Behälter, in dem die zweite Stufe des erfindungsgemäßen Verfahrens ausgeführt werden kann, kann beliebig ausgebildet sein. Im allgemeinen ist er feststehend angeordnet und enthält keine Einrichtungen für eine mechanische Durchmischung während der Evakuierung. Vorzugsweise ist der Behälter so konstruiert, daß das getrocknete Produkt auf einfache und schnelle Weise, beispielsweise am Boden, abgezogen werden kann. Hierzu kann es vorteilhaft sein, den Behälter mit einem schonend arbeitenden Rührorgan auszustatten. Um eine mögliche Kondensation von Nitrobenzol an den Behälterwänden zu verhindern, kann es vorteilhaft sein, sie beheizbar auszuführen. Die Trocknungszeit ist im allgemeinen sehr kurz, da normalerweise ein Restgehalt von Nitrobenzol von unter 1 Gew.-%, vorzugsweise unter 0,5 Gew.-%, schon dann vorliegt, wenn beim Evakuieren der Enddruck erreicht wird. Eine Staubentwicklung wird während der Evakuierung nicht beobachtet, so daß eine Filtration der Brüden nicht notwendig ist und ein gut rieselfähiges Produkt erhalten wird. Das getrocknete Produkt wird vorzugsweise nach Belüftung des Behälters aus diesem abgezogen.

Die zweite Stufe des erfindungsgemäßen Verfahrens wird vorzugsweise quasi-kontinuierlich betrieben, indem man vor dem evakuierbaren Behälter einen Pufferraum vorsieht und die Schritte »Füllen des Behälters«, »Evakuieren des Behälters«, »Belüften des Behälters« und »Entleerung des Behälters« in stetem Zyklus schnell hintereinander ablaufen läßt.

In einer bevorzugten Ausführungsform des erfindungsgemäßen Verfahrens befindet sich zwischen der ersten und zweiten Stufe eine Dosiervorrichtung, die über ein vakuumdichtes Absperrorgan mit dem evakuierbaren Behälter verbunden ist. Die Dosiervorrichtung kann auf verschiedene Weise ausgebildet sein. Vorzugsweise wird eine Schnecke verwendet. Die Dosiervorrichtung ist vorzugsweise beheizbar. Mit der Heizung kann entweder Wärmeverlust, z. B. durch Abstrahlung, kompensiert oder gegebenenfalls die zuvor beschriebene Temperaturerhöhung des Produktes aus der mechanischen Abtrennung durchgeführt werden. Die Temperaturerhöhung kann gegebenenfalls auch dadurch erfolgen, daß man den evakuierbaren Behälter vor oder während der Füllung erwärmt. Das vakuumdichte Absperrorgan kann beliebig ausgebildet sein, beispielsweise als Schieber.

Bei einer bevorzugten technischen Ausführungsform des erfindungsgemäßen Verfahrens wird wie folgt gearbeitet: Die Ausgangssuspension von festem 1,5- und/oder 1,8-Dinitroanthrachinon in flüssigem Nitrobenzol wird mit einer Temperatur im Bereich von 80 bis 150° C in ein Drucktrommelfilter oder eine Vollmantel-Schneckenzentrifuge eingegeben, in der bei konstanter Temperatur kontinuierlich flüssiges Nitrobenzol mechanisch abgetrennt und kontinuierlich ein Produkt mit weniger als 30 Gew.-% Nitrobenzolgehalt gewonnen wird. Gegebenenfalls wird in dem Drucktrommelfilter oder der Vollmantel-Schneckenzentrifuge auch noch eine Wäsche mit Nitrobenzol durchgeführt. Das abgetrennte Nitrobenzol wird entfernt. Das Produkt wird in einen Pufferraum ausgetragen, von dem es über eine beheizbare Schnecke und einen vakuumdichten Schieber in einen evakuierbaren Behälter gebracht wird. Mit der Schnecke wird eine bestimmte Menge des Produktes mit gleicher oder höherer Temperatur, wie sie das Produkt nach der mechanischen Abtrennung von Nitrobenzol aufweist, in den Behälter eindosiert. Nach Abstellen der Schnecke wird der Schieber geschlossen und der Behälter evakuiert. Das verdampfende Nitrobenzol wird vor der Vakuumpumpe abgeschieden. Nach Erreichen eines Enddrucks von 0,2 bis 30 mbar wird der Behälter belüftet und entleert. Danach beginnt der Zyklus von neuem.

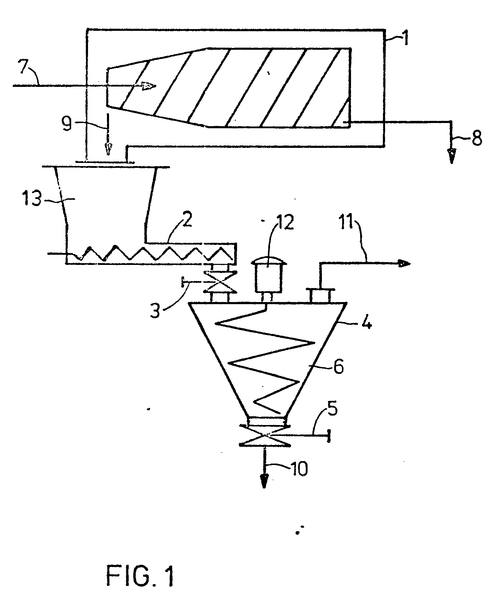

Eine besonders bevorzugte technische Ausführungsform des erfindungsgemäßen Verfahrens wird im folgenden anhand der Figur 1 erläutert:

Eine feste 1,5- oder 1,8-Dinitroanthrachinon enthaltende Nitrobenzol-Suspension (7) wird in eine mechanische Trennvorrichtung (1) eingebracht. Flüssiges Nitrobenzol wird über (8) entfernt, vorgetrocknetes 1,5- oder 1,8-Dinitroanthrachinon mit einer maximalen Restfeuchte von 30 Gew.-% (9) wird in den Pufferbehälter (13) ausgetragen. Über die Schnecke (2) und das Absperrorgan (3) wird das vorgetrocknete Produkt in den evakuierbaren Behälter (4) eindosiert. Nach Beendigung der Dosierung wird die Schnecke (2) abgeschaltet, das Absperrorgan (3) geschlossen und der Behälter (4) über Leitung (11) bis zu einem Enddruck von 1 bis 20 mbar evakuiert. Nach Erreichen des Enddrucks wird der Behälter (4) belüftet und mit Hilfe des Rührwerks (12) über den Ablaß (10) entleert. Die Temperatur des festen 1,5- oder 1,8-Dinitroanthrachinons wird bis zur Entfernung von Nitrobenzol im Vakuum im Bereich 90 bis 100°C gehalten.

Ein weiterer Gegenstand der vorliegenden Erfindung ist eine Vorrichtung zum Entfernen von Nitrobenzol aus Suspensionen, die festes 1,5- und/oder 1,8-Dinitroanthrachinon und Nitrobenzol enthalten, die dadurch gekennzeichnet ist, daß sie aus einer mechanischen Trennvorrichtung (1) und einem evakuierbaren Behälter (4) besteht. Vorzugsweise ist die mechanische Trennvorrichtung (1) ein Drucktrommelfilter oder eine Vollmantel-Schneckenzentrifuge und der evakuierbare Behälter (4) am Boden mit einer Ablaßvorrichtung (5) und mit einem Rührwerk (12) zur Erleichterung der Entleerung versehen.

Das erfindungsgemäße Verfahren und die erfindungsgemäße Vorrichtung haben eine Reihe von Vorteilen. So wird zur Verdampfung des Nitrobenzols die Wärme des Feststoffes in der Suspension ausgenutzt, und eine Wärmezufuhr während der Trocknung ist nicht notwendig. Auch wenn man die Dosiervorrichtung und/oder den evakuierbaren Behälter beheizt, sind hierfür nur geringe Wärmemengen erforderlich. Weiterhin bilden sich während der Trocknung keine staubförmigen Anteile, so daß ein gut rieselfähiges, nicht staubendes Produkt entsteht und Einrichtungen zur Filtration der Brüden wegfallen können. Wegen der kurzen Zeiten, die zur Trocknung benötigt werden, kann der evakuierbare Behälter relativ klein sein. Das getrocknete Produkt fällt im allgemeinen mit einer niedrigen Temperatur an, so daß es normalerweise so wie es anfällt abgepackt, gelagert oder zu weiteren Umsetzungen verwendet werden kann oder allenfalls nur eine mäßige Nachkühlung erforderlich ist.

Es ist überraschend, daß die erfindungsgemäße Trocknung auf so einfache Weise zum Erfolg führt. Übliche Vakuumtrockner beginnen mit der Trocknung bei relativ niedrigen Temperaturen, und durch Heizung während der Trocknung wird die Entfernung der letzten Feuchtigkeitsreste erleichtert. Beim erfindungsgemäßen Verfahren ist der Temperaturgang gerade umgekehrt, d. h., die letzten Feuchtigkeitsreste werden bei der tiefsten Temperatur entfernt. Trotzdem wird in kurzer Zeit ein trockenes Produkt erhalten. Weiterhin wird bei üblichen Vakuumtrocknern das Trockengut mechanisch durchmischt (Taumeltrockner, Schaufeltrockner), um den Wärmeübergang an das Produkt zu verbessern und das Herausdiffundieren von Feuchtigkeit aus den Feststoffteilchen zu erleichtern. Eine derartige mechanische Durchmischung ist beim erfindungsgemäßen Verfahren während der Trocknung im Vakuum nicht vorgesehen. Trotzdem wird in kurzer Zeit ein trockenes Produkt erhalten.

### Beispiele

#### Beispiel 1 (siehe auch Figur 1)

Die Abtrennung von 150 kg 1,8-Dinitroanthrachinon pro Stunde aus einem Dinitroanthrachinon-Isomerengemisch erfolgt durch Kristallisation in Nitrobenzol. Dabei fällt eine Suspension an, die 15 g festes 1,8-Dinitroanthrachinon pro kg der Suspension enthält. Die Suspension hat eine Temperatur von 107°C. Die flüssige Phase der Suspension enthält neben Nitrobenzol in gelöster Form isomere Dinitroanthrachinone.

Diese Suspension (7) wird einer beheizten Vollmantel-Schneckenzentrifuge (1) zugeführt. Darin wird aus der Suspension ein Großteil der flüssigen Phase entfernt und über (8) abgeleitet. Das feste 1,8-Nitroanthrachinon fällt mit einem Restfeuchtegehalt von 9 Gew.-% (9) an. Dieses wird mit einer Temperatur von 107°C in einem Pufferbehälter (13) gefördert und daraus über eine Dosierschnecke (2) und einen vakuumdichten Schieber (3) in einen evakuierbaren Behälter (4). Der evakuierbare Behälter wird mit einer Zykluszeit von 30 Minuten betrieben und hat einen Inhalt von 200 dm³. Nach dem Füllen wird die Dosierschnecke (2) abgeschaltet und der Schieber (3) geschlossen. Über die Leitung (11) wird der Behälter dann bis zu einem Vakuum von 5 mbar evakuiert.

Das 1,8-Dinitroanthrachinon trocknet dabei ohne Staubentwicklung auf Restfeuchten von unter 0,5 Gew.-% und kühlt sich dabei auf 70°C ab. Nach Erreichen des Drucks von 5 mbar wird der Behälter (4) belüftet und das getrocknete 1,8-Dinitroanthrachinon (10) mit Hilfe des Rührwerks (12) über den Ablaß (5) ausgetragen.

#### Beispiel 2 (siehe auch Figur 1)

500 kg 1,5-Dinitroanthrachinon pro Stunde werden durch Kristallisation in Nitrobenzol aus einem Dinitroanthrachinon-Isomerengemisch abgetrennt. Die 115°C heiße Suspension hat einen Gehalt von 110 g festem 1,5-Dinitroanthrachinon pro kg Suspension. Die flüssige Phase der Suspension enthält neben Nitrobenzol in gelöster Form isomere Dinitroanthrachinone. Diese Suspension wird über (7) in ein Drucktrommelfilter (1) eingegeben. Der größte Teil der flüssigen Phase wird in einem Drucktrommelfilter abgetrennt (8). Das feste 1,5-Dinitroanthrachinon (9) hat nach der mechanischen Entfeuchtung einen Restfeuchtegehalt von 15 Gew.-% und wird über einen kleinen Pufferbehälter (13) durch eine Dosierschnecke (2) in einen evakuierbaren Behälter (4) gefördert. Die Zykluszeit beträgt 30 min, der Behälter hat einen Inhalt von ca. 600 dm³. Während der Befüllung des Behälters wird über die auf 180°C beheizten Wände des Dosierorgans (2) das Feuchtgut auf 150°C aufgewärmt. Wie im Beispiel 1 wird nach dem Befüllen die Dosierschnecke abgestellt, der Behälter abgesperrt und evakuiert. Dabei trocknet das 1,5-Dinitroanthrachinon auf Endfeuchten von unter 0,5% und kühlt sich dabei auf 60 bis 70°C ab. Nach Erreichen des Drucks von 5 mbar wird der Behälter belüftet und das getrocknete 1,5-Dinitroanthrachinon (10) mit Hilfe eines Rührwerks (12) über den absperrbaren Auslaßstutzen (5) ausgetragen.

## Patentansprüche

1. Verfahren zur Gewinnung von trockenem 1,5- und/oder 1,8-Dinitroanthrachinon aus Suspensionen, die festes 1,5- und/oder 1,8-Dinitroanthrachinon und flüssiges Nitrobenzol enthalten, dadurch gekennzeichnet, daß man in einer ersten Stufe bei erhöhter Temperatur festes 1,5- und/oder 1,8-Dinitroanthrachinon mechanisch so aus der Suspension abtrennt, daß der Nitrobenzolgehalt weniger als 30 Gew.-% beträgt und in einer zweiten Stufe aus dem abgetrennten 1,5- und/oder 1,8-Dinitroanthrachinon restliches Nitrobenzol durch Druckerniedrigung bei sinkender Temperatur entfernt.

2. Verfahren nach Anspruch 1, dadurch gekennzeichnet, daß man die erste Stufe bei 80 bis 150°C durchführt.

3. Verfahren nach Anspruch 1 und 2, dadurch gekennzeichnet, daß das Produkt vor der Druckerniedrigung mit der gleichen Temperatur vorliegt wie nach der mechanischen Abtrennung von Nitrobenzol.

4. Verfahren nach Anspruch 1 und 2, dadurch gekennzeichnet, daß das Produkt vor der Druckerniedrigung auf eine Temperatur im Bereich 80 bis 205°C gebracht wird.

5. Verfahren nach Anspruch 1 bis 4, dadurch gekennzeichnet, daß der Druck auf 0,2 bis 30 mbar erniedrigt wird.

6. Verfahren nach Anspruch 1 bis 5, dadurch gekennzeichnet, daß während der Druckerniedrigung keine mechanische Durchmischung erfolgt.

7. Verfahren nach Anspruch 1 bis 6, dadurch gekennzeichnet, daß das Produkt aus der ersten Stufe über eine heizbare Dosiervorrichtung und ein vakuumdichtes Absperrorgan in die zweite Stufe eingebracht wird.

8. Verfahren nach Anspruch 1 bis 7, dadurch gekennzeichnet, daß man die erste Stufe kontinuierlich und die zweite Stufe quasi-kontinuierlich durchführt.

9. Vorrichtung zur Durchführung des Verfahrens nach Anspruch 1, dadurch gekennzeichnet, daß sie aus einer mechanischen Trennvorrichtung (1) und einem evakuierbaren Behälter (4) besteht.

10. Vorrichtung nach Anspruch 9, dadurch gekennzeichnet, daß die mechanische Trennvorrichtung (1) ein Drucktrommelfilter oder eine Vollmantel-Schneckenzentrifuge und der evakuierbare Behälter (4) am Boden mit einer Ablaßvorrichtung und mit einem Rührwerk (12) zur Erleichterung der Entleerung versehen ist.

## Claims

1. Process for obtaining dry 1,5- and/or 1,8-dinitroanthraquinone from suspensions which contain solid 1,5- and/or 1,8-dinitroanthraquinone and liquid nitrobenzene, characterised in that, in a first stage, solid 1,5- and/or 1,8-dinitroanthraquinone is separated off mechanically from the suspension at elevated temperature in such a way that the content of nitrobenzene is less than 30% by weight, and, in a second stage residual nitrobenzene is removed from the 1,5- and/or 1,8-dinitroanthraquinone which has been separated off by lowering the pressure as the temperature falls.

2. Process according to Claim 1, characterised in that the first stage is carried out at 80 to 150°C.

3. Process according to Claim 1 and 2, characterised in that, prior to lowering the pressure, the product is at the same temperature as after the mechanical separating off of nitrobenzene.

4. Process according to Claim 1 and 2, characterised in that, prior to lowering the pressure, the product is brought to a temperature in the range of 80 to 205°C.

5. Process according to Claim 1 to 4, characterised in that the pressure is lowered to 0.2 to 30 mbars.

6. Process according to Claim 1 to 5, characterised in that no mechanical mixing is carried out during lowering of the pressure.

7. Process according to Claim 1 to 6, characterised in that the product from the first stage is fed into the second stage via a heatable metering device and a vacuumtight shut-off device.

8. Process according to Claim 1 to 7, characterised in that the first stage is carried out continuously and the second stage is carried out quasi-continuously.

9. Equipment for carrying out the process according to Claim 1, characterised in that it consists of a mechanical separating device (1) and a vessel (4) to which a vacuum can be applied.

10. Equipment according to Claim 9, characterised in that the mechanical separating device (1) is a pressure drum filter or a complete cage screw centrifuge and the vessel (4) to which the vacuum can be applied is provided with an outlet device at the base and with a stirrer (12) to facilitate emptying.

**Revendications**

1. Procédé pour l'obtention de 1,5- et/ou de 1,8-dinitroanthraquinone sèche à partir de suspensions qui contiennent de la 1,5- et/ou 1,8-dinitro-anthraquinone solide et du nitrobenzène liquide, caractérisé en ce que, dans une première étape à température élevée, on sépare mécaniquement la 1,5- et/ou la 1,8-dinitro-anthraquinone solide de la suspension de manière que la teneur en nitrobenzène s'élève à moins de 30% en poids et, dans une seconde étape, on élimine le nitrobenzène résiduel de la 1,5- et/ou de la 1,8-dinitro-anthraquinone séparée, par abaissement de la pression avec décroissance de la température.

2. Procédé suivant la revendication 1, caractérisé en ce qu'on conduit la première étape à 80–150°C.

3. Procédé suivant l'une des revendications 1 et 2, caractérisé en ce que le produit présente avant l'abaissement de pression la même température qu'après la séparation mécanique du nitrobenzène.

4. Procédé suivant l'une des revendications 1 et 2, caractérisé en ce que le produit est porté à une température comprise dans la plage de 80 à 205°C avant l'abaissement de pression.

5. Procédé suivant l'une des revendications 1 à 4, caractérisé en ce que la pression est abaissée à 0,02 à 3 kPa.

6. Procédé suivant l'une des revendications 1 à 6, caractérisé en ce qu'aucune action mécanique de mélange n'est effectuée pendant l'abaissement de pression.

7. Procédé suivant l'une des revendications 1 à 6, caractérisé en ce que le produit venant de la première étape aborde la seconde étape en passant par un dispositif doseur pouvant être chauffé et par un organe d'arrêt étanche au vide.

8. Procédé suivant l'une des revendications 1 à 7, caractérisé en ce que la conduite de la première étape est continue et la conduite de la seconde étape est quasi continue.

9. Dispositif pour la mise en oeuvre du procédé suivant la revendication 1, caractérisé en ce qu'il est constitué par un séparateur mécanique (1) et par un récipient (4) dans lequel le vide peut être crée.

10. Dispositif suivant la revendication 9, caractérisé en ce que le séparateur mécanique (1) est un tambour filtrant fonctionnant sous pression ou une centrifugeuse à vis sans fin à bol plein et le récipient (4) pouvant être vidé d'air présente au fond un dispositif de vidange et est pourvu d'un agitateur (12) pour faciliter le vidage.

FIG. 1